# EUROPEAN PATENT SPECIFICATION

(11) **EP 1 242 602 B1**
(45) Date of publication and mention of the grant of the patent: **13.02.2008**
(21) Application number: 00983103.3
(22) Date of filing: 02.11.2000
(51) Int. Cl.: C12N 15/79

(54) **PROTEIN EXPRESSION SYSTEMS IN NON-PATHOGENIC KINETOPLASTIDAE**
NICHT-PATHOGENES KINETOPLASTID PROTEIN-EXPRESSIONSSYSTEM
SYSTEMES D'EXPRESSION DE PROTEINES DANS KINETOPLASTIDAE NON PATHOGENES

(30) Priority: 05.11.1999 EP 99122222
(43) Date of publication of application: 25.09.2002
(73) Proprietor: Jena Bioscience GmbH, 07749 Jena (DE)
(72) Inventor: ALEXANDROV, Kirill, 44141 Dortmund (DE); GRUN, Mathias, Somerville, MA 02144 (US)
(74) Representative: Ackermann, Joachim
(86) International application number: PCT/EP2000/010794
(87) International publication number: WO 2001/032896

(56) References cited:
- EP-A- 0 939 130
- WO-A-00/32796
- WO-A2-00/58483
- ZHANG W W ET AL: "The expression of biologically active human p53 in Leishmania cells: a novel eukaryotic system to produce recombinant proteins." NUCLEIC ACIDS RESEARCH, (1995 OCT 25) 23 (20) 4073-80., XP002147127 cited in the application
- PATENT ABSTRACTS OF JAPAN vol. 012, no. 176 (C-498), 25 May 1988 (1988-05-25) & JP 62 282585 A (SANRAKU INC), 8 December 1987 (1987-12-08)
- CLAYTON C.E.: 'Genetic manipulation in Kinetoplastida' PARASITOLOGY TODAY vol. 15, no. 9, September 1999, ESSEX, GB, pages 372 - 378, XP002175993
- KUSHNIR ET AL PROTEIN EXPRESSION AND PURIFICATION vol. 42, pages 37 - 46
- GEORGIOU G.; SEGATORI L. CURRENT OPINION IN BIOTECHNOLOGY vol. 16, pages 538 - 545
- GELLISSEN G. ET AL FEMS YEAST RESEARCH vol. 5, 2005, pages 1079 - 1096

### SEQUENCE LISTING

<110> Jena Bioscience GmbH
<120> Protein expression systems for non-pathogenic Kinetoplastidae
<130> 199jb01.wo
<140>
   <141>
<150> 99122222.5
   <151> 1999-11-05
<160> 7
<170> PatentIn Ver. 2.1
<210> 1
   <211> 43
   <212> DNA
   <213> Artificial Sequence
<220>
   <223> Description of Artificial Sequence: Primer
<400> 1
   ctgcagtgat cagtcgccac catgcggggt tctcatcatc atc 43
<210> 2
   <211> 38
   <212> DNA
   <213> Artificial Sequence
<220>
   <223> Description of Artificial Sequence: Primer
<400> 2
   ctgcagtgat caagatcttc actcggcagg cgggggac 38
<210> 3
   <211> 19
   <212> DNA
   <213> Artificial Sequence
<220>
   <223> Description of Artificial Sequence: Primer
<400> 3
   ctgcaccgtg gtcgactgc 19
<210> 4
   <211> 24
   <212> DNA
   <213> Artificial Sequence
<220>
   <223> Description of Artificial Sequence: Primer
<400> 4
   cctagtatga agatttcggt gatc 24
<210> 5
   <211> 22
   <212> DNA
   <213> Artificial Sequence
<220>
   <223> Description of Artificial Sequence: Primer
<400> 5
   ctgcaggttc acctacagct ac 22
<210> 6
   <211> 22
   <212> DNA
   <213> Artificial Sequence
<220>
   <223> Description of Artificial Sequence: Primer
<400> 6
   ggctagttag gcgtcatcct ga 22
<210> 7
   <211> 22
   <212> DNA
   <213> Artificial Sequence
<220>
   <223> Description of Artificial Sequence: Primer
<400> 7
   ggccgattca ttaatgcagg ac 22

## Description

The invention relates to *L. tarentolae* host cells useful in the production of recombinant proteins. In particular, the invention relates to *L. tarentolae* host cells, which can be used in the high-level expression of recombinant proteins, especially enzymes.

### Background of the invention:

Recombinant technology was successfully employed to transfer heterologous DNA coding for genes into a number of pro- and eukaryotes. Currently, there is a selection of expression systems to choose from for the production of any given protein, including prokaryotic and eukaryotic hosts. The selection of an appropriate expression system will often depend not only on the ability of the host cell to produce adequate yields of the desired protein in an active state, but also to a large extent may be governed by the intended use of said protein.

Genomic or episomal integration of the gene for the desired protein accompanied by adequate elements such as promoters, polyadenylation sites, 3' and 5' untranslated regions (UTRs) can mediate heterologous gene expression. This technology was successfully applied to a number of cell types such as bacteria, yeast, filamentous fungi, cultures of cells or entire insect and mammalian organisms. Each of these expression systems is associated with specific utilities and shortcomings in terms of protein yield, quality and cost and no universal host for protein expression exists.

A protein expression host has to satisfy several criteria:

The host should be easily cultivatable on cheap media and easily manipulatable by standard molecular biological procedures. Furthermore, the host should produce active proteins in high quantities and allow the extraction of the latter.

The *Kinetoplastidae* are primitive flagellated protozoans found in terrestrial and aquatic environments. Some of them cause diseases in organisms ranging from plants to vertebrates. Evolutionary position of *Kinetoplastidae* and systematic structure of the group are described elsewhere (e.g. Adoutte and Philippe, 1993). Two major sub-groups of *Kinetoplastidae* are *Leishmania* and *Trypanosomatidae. Kinetoplastidae* have been particularly valuable for the study of fundamental molecular and cellular phenomena, such as RNA editing (Stuart, 1991), mRNA trans-splicing (Perry and Agabian, 1991), glycosylphosphatidylinositol-anchoring of proteins (Krakow et al., 1986), antigenic variation (Borst and Rudenko, 1994), and telomer organization (Blackburn, 1991). Another unique among eukaryotes feature of *Trypanosomatidae* are polycistronic transcription units. In these units, series of genes are arranged in tandem structures and transcribed by RNA polymerase as a single 50-100 kb transcript (Myler et al., 1999; Teixeira, 1998). Such transcript is then processed into single gene mRNAs by addition of capped mini-exon at the 5' end by a process known as a trans-splicing followed by polyadenylation and cleavage of the 3' end. Gene expression and its control in *Kinetoplastidae* appears to deviate significantly from those of other eukaryotes. In few cases e.g. *Trypanosoma brucei,* stage specific promoters such as VSG and PARP promoters were identified (Barry et al., 1998; Hotz et al., 1998). In contrary to all other described so far eukaryotes these promoters recruit RNA polymerase I and not RNA polymerase II for protein encoding genes (Teixeira, 1998). In the case of *Leishmania* species with an exemption of ribosomal RNA promoter no elements that would resemble a promoter were identified and according to a current model initiation of transcription in *Leishmania* by RNA polymerase II is a random process. As a result of such gene organization most of gene regulation occurs on a post transcriptional level (Teixeira, 1998).

From the early nineties a significant progress was made towards establishment of reversed genetics methodology for *Kinetoplastidae* research. For the first time, vectors were reported that allowed transient expression of foreign genes in *Trypanosoma cruzi* (Lu and Buck, 1991). Supplying the transfer vector with a NEO gene as drug resistance marker transformation was made stable and successively reported for several other species (Coburn et al., 1991). Transfer of DNA constructs was accomplished by electroporation or particle bombardment (Beverley and Clayton, 1993; Sbicego et al., 1998). It was shown that *Kinetoplastidae* are able to replicate both, foreign and genome derived plasmids (Coburn et al., 1991; Patnaik et al., 1994; ten Asbroek et al., 1993) (Papadopoulou et al., 1994). It was demonstrated that one or both alleles of a functional gene could be deleted by homologous recombination and new genes could be integrated into the genome (Cruz et al., 1991; Ray and Hines, 1995). A range of methods was developed that allowed expression of cytoslic or secreted proteins in *Leishmania* and *Trypanosomatidae.* These constructs could be roughly divided in to three categories:
A) Episomal plasmids carrying no defined promoters in the case of *Leishmania* or stage specific promoters in the case of *Trypanosoma bruci* (LeBowitz et al., 1990)(La Flamme et al., 1996)
B) Constructs integrated into actively transcribed gene clusters (tubulin genes, small subunit ribosomal RNA genes, large subunit ribosomal RNA genes )(Misslitz A. et al., 1999; Tobin and Wirth, 1992; Wirtz et al., 1999).
C) Integration of foreign polymerase genes into an actively transcribed locus and transcription of the gene of interest by this polymerase (especially T7 or T3 polymerase ) (Wirtz et al., 1994) .

Using those approaches several genes were expressed and their products were demonstrated to be functionally active:
- p53 in *Leishmania donovani* (Zhang et al., 1995)
- Interferon-gamma in *Leishmania major* (Tobin and Wirth, 1992) .
- Luciferase in *Trypanosoma brucei* (Biebinger et al., 1997).
- T7 and T3 polymerase in *Trypanosoma brucei* (Wirtz et al., 1994)
- Therapeutic proteins in *Leishmania major* (WO 00/58483)

The prior art also includes publications concerned with methods for *in vitro* cultivation of a number of *Kinetoplastidae* including growth on solid (plates) and liquid media (flasks or fermenters) (Alfonzo et al., 1998). In most cases the media contained a selection of essential amino acids and microelements supplemented with bovine calf serum or bovine blood (Beverley and Clayton, 1993; Hill and Fahey, 1987). Several species were demonstrated to grow in the absence of serum on the simple non-synthetic media. The best described and studied of those species as *Leishmania tarentolae Parrot* (parasite of gecko), *Tarentola mauritanica* and *Tarentola annularis* (Elwasila, 1988); or *Crithidia fasciulata Leger* parasite of mosquito *Culex pipiens* (Leger 1902 ) could be cultivated on brain heart infusion medium (BHI) (Alfonzo et al., 1998) or Luria-Bertani medium (LB). These species were also demonstrated to grow well in large fermenters. *Leishmania tarentolae* could reach the density of 2x10⁸ cells per ml in the late stationary phase in 15 L standard fermenters with the yield of 10g of dry cell weight per 1L of culture (Alfonzo et al., 1998).

However, other species of *Kinetoplastidae* - especially species of *Trypanosomatidae* and *Leishmania -* have not been shown to be useful in expression of heterologous proteins, due to associated health risks, low growth rates or uncommon, expensive culture mediums.

An ideal expression and delivery system is one which is substantially free of production of proteases, toxins and large amounts of other endogenously synthesized and secreted proteins of the host, and which is capable to reach high protein expression levels.

In the prior art all of the experiments and investigations were carried out with pathogenic species *in vitro or in vivo* and no attempts were made to isolate the recombinant proteins.

Experiments showed, that the desired heterologous protein expression can be established with non pathogenic species of *Kinetoplastidae,* in particular from *Leishmania tarentolae, Crithidia fasciculata, Wallaceina inconstans (former Proteomonas inconstans), Leptomonas collos, Leptomonas sp. Cfm, Leptomonas sp. Nfm or Leptomonas seymouri.*

The above described species showed superior growth rates on cheap media when compared to other *Kinetoplastidae* and therefore demonstrated the utility as hosts for protein production.

The object of the present invention is therefore to provide a new expression and delivery system and / or protein expression host of non pathogenic *Kinetoplastidae,* which can be used in the high-level expression of recombinant proteins, especially enzymes.

The object is achieved in accordance to claim 1 by non pathogenic *Leishmania tarentolae* host cells comprising a nucleic acid sequence encoding a heterologous protein.

### Especially by:

*Leishmania tarentolae Parrot* ATCC number 30143 and *Crithidia fasciculata Leger* ATCC number 12857 (American Type Culture Collection number).

By heterologous protein is meant a protein which is not native to the host cell, or a native protein in which modifications have been made to alter the native sequence. In a preferred embodiment the protein is a heterologous enzyme. Therefore a coding heterologous nucleic acid sequence is operably linked to a suitable promoter sequence and, if appropriate, to post transcriptional signal sequences, which are capable of directing expression of the nucleic sequence in the chosen host cells.

Organisms of the order *Kinetoplastidae,* like the non pathogenic species *Leishmania tarentolae, Crithidia fasciculata, Wallaceina inconstans, Leptomonas collos., Leptomonas sp. Cfm, Leptomonas sp. Nfm* or *Leptomonas seymouri,* have a unique organelle called the kinetoplast, an appendix of their single mitochondrion located near the basal body of the flagellum that contains a network of thousands of small interlocking circular DNAs. *Kinetoplastidae* have a size of 50-400 microns. *Kinetoplastidae* are among the most ancient eukaryotes, with rRNA lineage extending further back than those of animals, plants, and even fungi (Beverley, 1996). *Kinetoplastidae* species parasite in a very diverse spectrum of hosts ranging from plants to humans. Many representatives of *Kinetoplastidae* were isolated and maintained in culture for an extended period of times. Cultivation could be carried out on both, completely or partially defined liquid and solid media (Melo, 1981). *Kinetoplastidae* grown in a liquid media, form suspensions whereas plating on solid media leads to formation of colonies (Hill and Fahey, 1987).

For the purpose of the present invention, "non pathogenic" is defined by classification of the organisms in questions to the Biosafety Level 1 according to the guidelines of the U.S. department of Health and Human Servies (HHS Publication No. (CDCD 93-8395) or at http://www.niehs.nih.gov/odhsb/biosafe/bio.htm.

The transformation of the selected species are conducted by using amounts of DNA ranging between 1-100 µg and selection with an adequate plating techniques and conditions for an antibiotic selection or with a limited dilution techniques. Transformation efficiency ranges broadly depending on the species chosen, being the highest for *Leishmania* species and approaching 10⁻⁴/cell (Kapler et al., 1990), (Coburn et al., 1991). A single cell can maintain several expression constructs provided that they all carry different selection markers (Wirtz et al., 1999). Levels of expression vary significantly depending on the host and construct chosen with episomal plasmids being on the low end of the scale but nevertheless able to generate recombinant protein up to 1% of total cellular protein (LeBowitz et al., 1990).

The results show clearly, the selected pathogenic species are capable of expressing and secreting a heterologous protein. Thus, it is understood that this ability is not limited to a single strain, but rather is a characteristic of this group of species as a whole. Those skilled in the art will recognize that other strains of these species can also be used in expression of heterologous proteins. Many strains are publicly available. For example *Leishmania tarentolae Parrot* ATCC number 30143 and *Crithidia fasciculata Leger* ATCC number 12857.

The skilled person in the art will also recognize that the successful transformation of the host species described herein is not limited to the use of the vectors, promoters and selection markers specifically exemplified. Those techniques are also implicated, which are useful in connection with the *L. tarentolae* host cells of the present invention.

The term "useful" includes but is not limited to techniques comprising a hygromycin phosphotransferase (HYG) gene in combination with hygromycin, neomycin phosphotransferase (NEO) gene in combination with G418, the product of the *Streptoalloteichus hindustanus* BLE gene (BLE) in combination with phleomycin and streptothricin acetyltransferase encoding (SAT) gene in combination with nourseothricin which are used for selection of recombinant clones. Additionally it includes but is not limited to the 5' and 3' intergenic regions of actively transcribed genes of the host such as calmodulin, dihydrofolate reductase-thymidylate synthase and cystein proteinase genes.

The invention also provides a vector, which is capable to transform non pathogenic *Kinetoplatidae,* in particular the said *L. tarentolae* species, wherein the nucleic acid is coding for a desired protein flanked by 5'and 3'UTR of an actively transcribed *L*. *tarentolae* gene, including signals for efficient trans-splicing and polyadenylation and which is containing, if appropriate, a selection marker.

The vector system may be a single vector or plasmid or a system of two or more vectors or plasmids, which together contain the total DNA to be transferred into the host cell. The vectors or plasmids may be linear or closed circular molecules.

According to a preferred embodiment of this invention, the vector system preferably comprises a linearized plasmid nucleic acid and a heterologous nucleic acid coding for a protein of interest flanked by intergenic regions of an actively translated host protein operably linked to a resistance marker gene flanked by segments of an actively transcribed host gene. Such genes include but are not limited to small subunit ribosomal RNA genes, large subunit ribosomal RNA genes, α or β-tubulin genes, the calmodulin gene or the gp63 gene. The construct is integrated into the actively transcribed clusters such as small subunit ribosomal RNA gene cluster, large subunit ribosomal RNA gene cluster, alpha or beta-tubulin gene cluster by homologous recombination and selection with the appropriate antibiotic.

According to another preferred embodiment of this invention, the vector system preferably comprises a linearized plasmid nucleic acid and heterologous nucleic acid coding for a protein of interest flanked by intergenic regions of an actively translated host protein operably linked to a resistance marker gene and flanked by fragments of DNA from an untranslated spacer of the rRNA gene cluster. The gene coding for the protein of interest is prefaced with the ribosomal RNA gene promoter and in some cases with regulatory elements including but not limited to Tet or Lac repressor responsive elements. The construct is integrated into an untranscribed spacer of the rRNA gene region by homologous recombination and selection with the appropriate antibiotics.

According to another preferred embodiment of this invention, the vector system preferably comprises an episomally available circular plasmid nucleic acid and a heterologous nucleic acid coding for a protein of interest flanked by intergenic regions of an actively translated host protein operably linked to a resistance marker.
The construct is maintained in the organism episomally under the pressure of antibiotic selection and transcripts can be generated by random initiation and run-around transcription.

According to another preferred embodiment of this invention, the vector system preferably comprises an episomally available circular plasmid nucleic acid and heterologous nucleic acid coding for a protein of interest flanked by intergenic regions of an actively translated host protein operably linked to a resistance marker. The nucleic acid coding for the gene of interest is prefaced with either a ribosomal promoter and its regulatory elements or with a promoter for a foreign RNA polymerase including but not limited to T7 , T3, etc. The construct is maintained in the organism episomally under the pressure of antibiotic selection and transcripts are generated by RNA polymerase I in the case of the ribosomal RNA gene promoter. In the case of other promoters (T7, T3 etc) transcription is driven by foreign polymerase integrated into the genome.

According to another preferred embodiment of the present invention, the host is transformed with three vectors, one including the gene for a foreign RNA polymerase (for example T7 or T3 polymerase) with intergenic regions of an actively expressed host protein operably linked to a resistance marker gene and flanked by segments of an actively transcribed host gene. This construct is integrated into an actively transcribed gene cluster of the host by homologous recombination.

The other plasmid comprises the heterologous DNA coding for a Tet repressor with intergenic regions of an actively expressed host protein and includes a promoter, preferably T3 or attenuated T7 promoter in front of it. This modified Tet repressor gene is operably linked to an antibiotic resistance marker gene and the entire construct is flanked by segments of an actively transcribed host gene.This construct is integrated into the actively transcribed gene cluster of the host by homologous recombination.

The third vector comprises an episomally available plasmid containing the heterologous DNA for the protein of interest to be expressed, flanked by intergenic regions of an actively expressed host protein and bearing preferably a T3 or T7 specific promoter at the 5' end of the above indicated gene for the desired protein. This promoter is additionally supplied with the TET repressor recognition sequence to allow for tetracycline dependent control of transcription. The resulting construct is operably linked to an antibiotic resistance marker gene and maintained episomally or supplied with appropriate target sequences flanking the entire construct and integrated into a transcriptionally silent part of the host genome such as the non-transcribed rRNA gene spacer.

The present host cell species can be used to express any prokaryotic or eukaryotic heterologous protein of interest, and is preferably used to express eukaryotic proteins. For example, the novel expression and delivery systems can be used to express enzymes such as catalase, laccase, phenoloxidase, oxidase, oxidoreductases, cellulase xylanase, peroxidase, lipase, hydrolase, esterase, cutinase, protease and other proteolytic enzymes, aminopeptidase, carboxypeptidase, phytase, lyase, pectinase and other pectinolytic enzymes, amylase glucoamylase, α-galactosidase, β-galactosidase, α-glucosidase, β-glucosidase, mannosidase, isomerase, invertase, transferase, ribonuclease, chitinase, and deoxyribonuclease. It will be understood by those skilled in the art that the term "enzymes" includes not only native enzymes, but also those enzymes which have been modified by amino acid substitutions, deletions, additions, or other modifications which may be made to enhance activity, thermostability, pH tolerance etc.

The present host cells may also be used in the recombinant production of proteins which are native to the host cells. Examples of such use include, but are not limited to, placing an non pathogenic *Kinetoplastidae* -type native protein under the control of a different promoter to enhance expression of the protein, to expedite export of a native protein of interest outside the cell by use of a signal sequence, or to increase the number of copies of a protein which is normally produced by the subject host cells. Thus, the present invention also encompasses such recombinant production of homologous proteins, to the extent that such expression involves the use of genetic elements not native to the host cell, or use of native elements which have been manipulated to function in a manner not normally observed in the host cell.

To avoid the necessity of disruption of the cells to obtain the expressed product, and to minimize the extend of possible degradation of the expressed product within the cells, it is preferred that the product is secreted out of the cells. In a preferred embodiment, the gene of interest is fused to the DNA sequence for a pre-region such as a signal or leader peptide which can mediate the expressed product into the cell's secretory pathway. The pre-region may be derived from genes for any secreted protein from any organism, or preferred is a native pre-region of the selected species or of the selected protein. It can include but is not limited to a secretory signal sequence of mammalian interferon-gamma, secretory signal sequence of secreted acid phosphatase from *Leishmania mexicana,* secretory signal sequence of soluble acid phosphatase from *Leishmania donovani,* secretory signal sequence of *L. tarentolae* 9P63 gene or other secretory signals of other secreted proteins.

The invention provides also stably transformed *L. tarentolae* cell-lines obtainable by the disclosed vector and vector system.

Further features of the present invention are more detailled described in the following Figures and Examples:

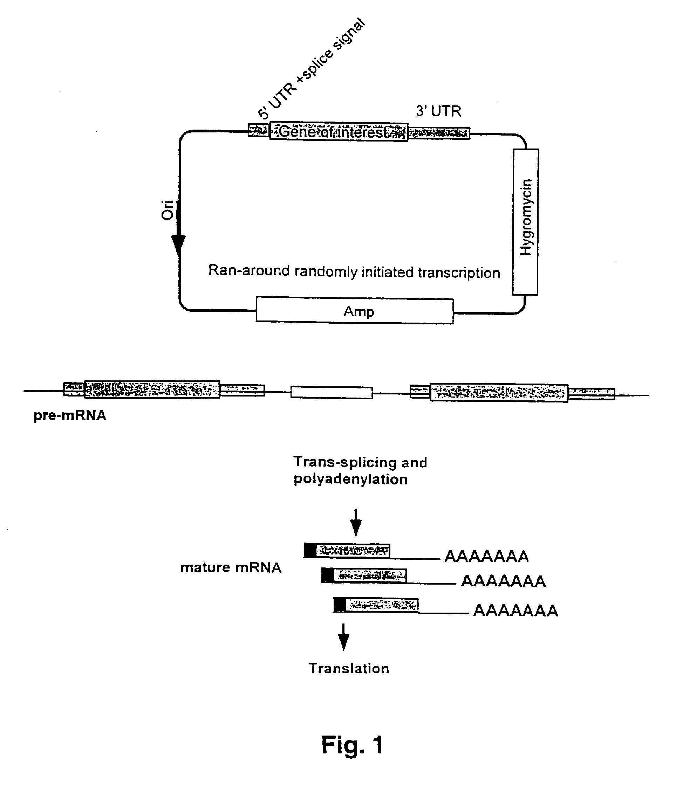

Figure 1: Protein expression from episomal vectors in *Kinetoplastidae.*

Schematic representation of an episomal plasmid vector for heterologous gene expression in *Kinetoplastidae* species. The plasmid contains a prokaryotic origin of replication and a prokaryotic resistance marker gene (Amp). The gene of interest is flanked by intergenic regions of the host and an eukaryotic selection marker gene (Hyg). Transcription is initiated randomly and generates transcripts of random length.

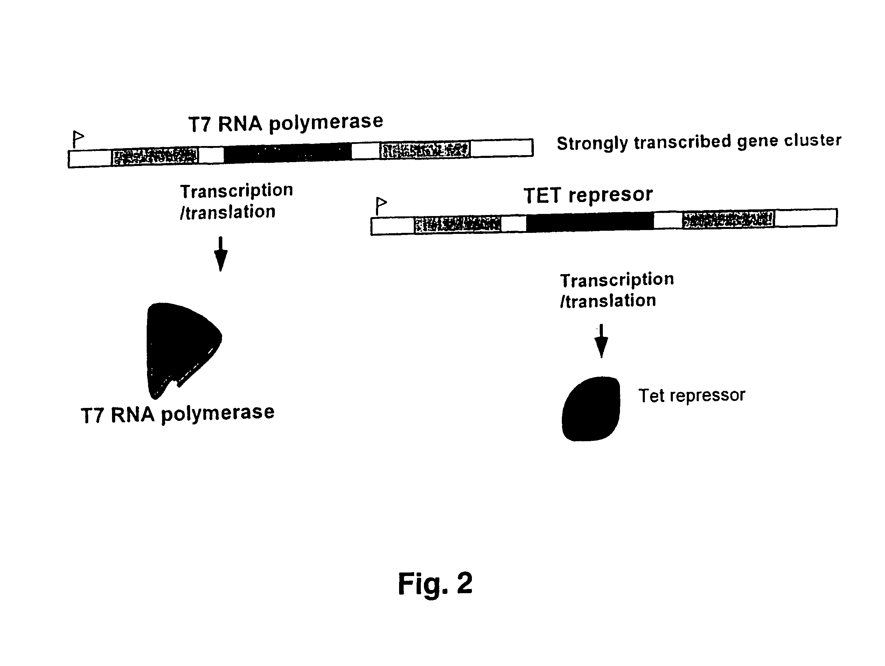

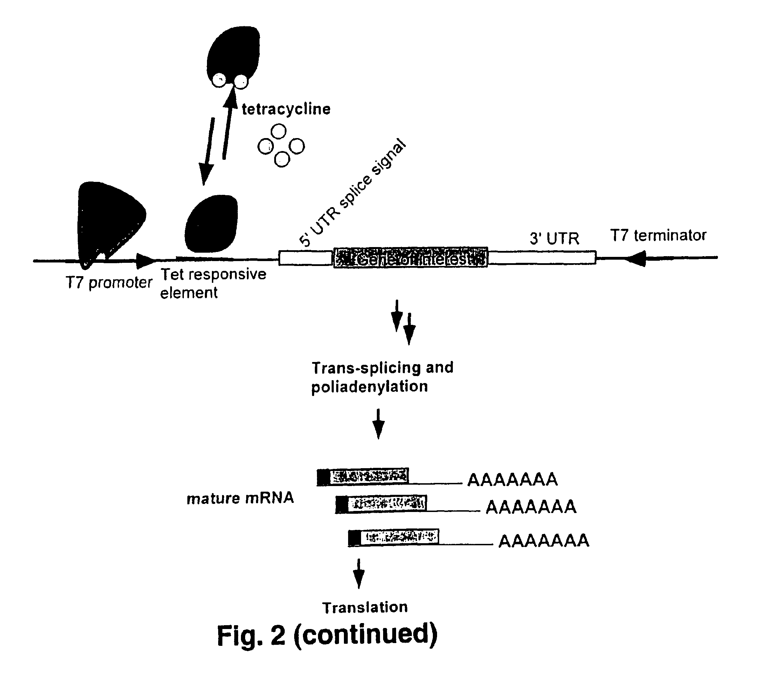

Figure 2: RNA polymerase mediated protein expression in *Kinetoplastidae.*

Schematic representation of heterologous gene expression in *Kinetoplastidae* species based on a phage polymerase and controlled by a Tet responsive element. T7 RNA polymerase and Tet repressor genes are integrated into an actively transcribed locus, preferred into an strongly transcribed gene cluster. The gene of interest is integrated into the transcriptionally silent region of the genome under the control of T7 promoter and Tet responsive element. Transcription of the gene of interest is initiated by addition of tetracycline to the culture medium.Trans-splicing, polyadenylation and translation of the mature mRNA into the protein of interest is following.

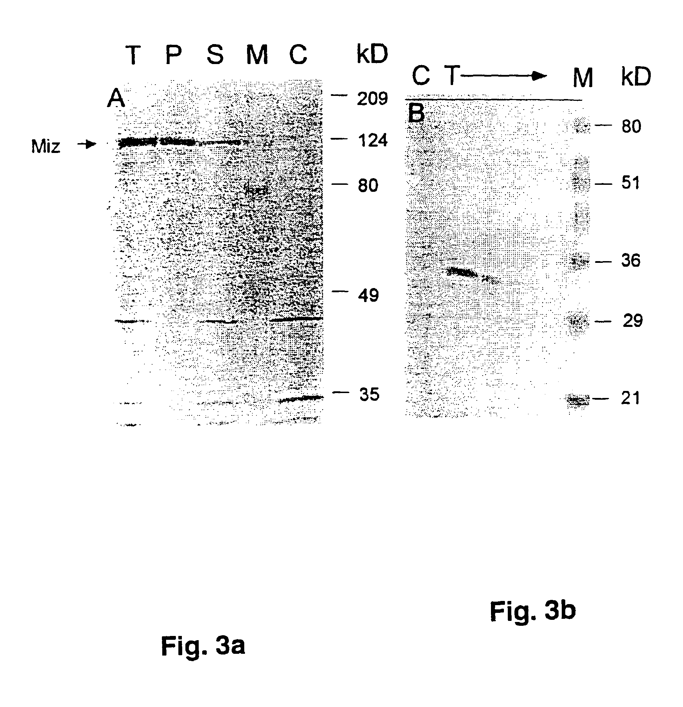

Figure 3: Western blot of *Leishmania tarentolae* cells expressing proto-oncogen Miz-1 (A) and human erythropoetin (B).

Western blot of *Leishmania tarentolae* cells expressing proto-oncogen Miz-1 (A) Cells transformed with the pIR-miz construct were selected for antibiotic resistance and expended in the BHI broth containing 100mg/L nourseothricin. Cells were harvested by centrifugation and either directly boiled in the Laemmli buffer or were first partitioned into soluble and insoluble fractions with Triton X-100. The lysates were separated on the 10% SDS-PAGE, transferred onto the nitro-cellulose and recombinant Miz was probed with polyclonal anti-miz-1 antibody.
Column T (Fig. 3 A) show the total lysate of *L. tarentolae* cells expressing miz. Column P (Fig. 3 A) show the 1% TritonX-100 insoluble fraction of *L. tarentolae* cells expressing miz.
Column S (Fig. 3 A) show the 1% TritonX-100 soluble fraction of *L. tarentolae* cells expressing miz.
Column M show the molecular weight markers, Column C show the lysate of control cells.

Western blot of cultural supernatants of *Leishmania tarentolae* cells expressing human erythropoetin (B). For erythropoetin expression 200 microliters of the cultural supernatants were precipitated with 10% TCA (Tri-Chlor-Acetic acid) and separated on the 15% SDS-PAGE , transferred onto the nitro-cellulose and recombinant erythropoetin was probed with polyclonal anti- erythropoetin antibody.
The columns T (Fig. 3 B) show the different amounts of cultural supernatant of *L*. *tarentolae* cells expressing human Epo.
Column M show the molecular weight markers, Column C show the lysate of control cells.

The following examples are not covered by the claims, but may serve as proof of principle, without restricting the same to the products and embodiments described in the examples.

### Example 1.

### Expression of proto-oncogene Miz-1 in Leishmania tarentolae

Cloning of the *miz*-1gene into the pIR vector:
The pIR vector (Hubel A. and Beverley, 1999){Wirtz, Leal, et al. 1999 ID: 267} containing
- the ColE1 origin for replication and ampicillin resistance gene (Amp) for plasmid propagation and selection in *E*. *coli,*
- a BgIII site for insertion of gene cassettes, flanked by intergenic regions of cys2 (cysteine proteinase) and LPG1 (glycosyltransferase) genes of *Leishmania* with signals for trans-splicing and polyadenylation of the mRNA of the gene for the desired protein,
- the streptothricin acetyltransferase (sat) gene from transposon Tn7, flanked by intergenic regions of LPG1 and DHFR-TS (dihydrofolate reductase-thymidylate synthase) genes of *Leishmania* conferring resistance of *Leishmania* to the antibiotic nourseothricin,
- two segments of approximately 1 kbp (a 5' and a 3' part) of the gene for the small ribosomal subunit RNA *(ssu)* of *Leishmania,* bracketting the expression signals linked to the sat resistance marker, and enabeling integration of the DNA in between the ssu-boxes into the genome of *Leishmania tarentulae* by homologous recombination following cleavage of recombinant pIR vector with restriction enzyme Smil.

The coding sequence of Miz-1 (gene bank accession Nr. Y09723)was amplified by PCR according to standard procedures from a plasmid source (pFH50) supplying the genetic information for an extra hexahistidine tag at the N' terminus of the Miz-1 protein. Following primers were used introducing recognition sequences for the restriction enzyme Bcl I at both ends of the 2.6 kbp PCR fragment :
- F2460 miz-1 forward primer: CTG CAG TGA TCA GTC GCC ACC ATG CGG GGT TCT CAT CAT CAT C (Seq. ID No. 1, start codon underlined) and
- F2461 miz-1 reverse primer: CTG CAG TGA TCA AGA TCT TCA CTC GGC AGG CGG GGG AC (Seq. ID No. 2, stop codon underlined).

Standard molecular cloning techniques were applied to insert the *miz-1* gene into the vector pIR. The ends of the PCR fragment were first trimmed with Bcll and the PCR product was than ligated with vector pIR linearized with BgIII. Following transformation *of E. coli* TG1 with the ligation mixture the clones containing recombinant pIR with *miz-1* insert in the correct orientation (pIRmiz1) were identified by colony-PCR using primers F2718 (pIR-BgIII-forward) CTG CAC CGT GGT CGA CTG C (Seq. ID No. 3), annealing upstream of the BgIII site of pIR and primer F2461 (miz-1 reverse, described earlier). Plasmid DNA was extracted from positive clones and the correct structure and sequence of pIRmiz1 was confirmed by restriction analysis and sequencing. Large scale plasmid preparations were achieved with the Plasmid Maxi Kit (Qiagen).

Transformation of *Leishmania tarentolae* with plRmiz1:
For chromosomal integration of the *miz-1* gene into the *ssu*-cluster approx. 10 µg pIRmiz1 were cleaved with restriction enzyme Smil, resuspended at 0.5 µg/µl and introduced into *Leishmania tarentolae* cells by electroporation.
Electroporation was conducted with a Multiporator (Eppendorf) according to the protocol of the supplier. Per transformation 1.0 ml of a growing *L. tarentolae* culture in BHI broth with 5 µg/ml hemin (OD₆₀₀ = 1.0) were harvested by centrifugation (1 min at 5000 x g), washed once in hypoosmolaric buffer (HOP) (Eppendorf cat.-No. 4308 070 501) and resuspended in 1.0 ml HOP. Per transformation 0.4 ml of this cell suspension were used. The cells were kept on ice for 10 min, DNA was added and electroporation was carried out in a cuvette d=2 mm at 1000 V and 160 µsec. Following the pulse the cells were kept on ice for 10 min resuspended in 10 ml BHI with 5 µg/ml hemin and incubated at 26°C. Selection with 100 µg/ml nourseothricin was applied after 20 h and recombinant lines were selected by limited dilution. The expected chromosomal structure of the recombinant *L. tarentolae* ::pIAmiz1 cells (integration of the expression unit with the heterologous *miz-1* gene into the ssu cluster) was confirmed by PCR diagnostics of genomic DNA of these cells with three specific primer pairs. The first primer pair consisted of sat forward primer F2999 (CCT AGT ATG AAG ATT TCG GTG ATC) (Seq. ID No. 4) annealing inside of the recombination region *(sat* gene of pIR) and ssu reverse primer F3002 (CTG CAG GTT CAC CTA CAG CTA C) (Seq. ID No. 5) annealing outside of the recombination region (3' *ssu* gene region not present on pIR) and generated a characteristic 2.3 kbp fragment missing in the wild type control. The other two primer pairs were specific for the integrated segment of pIRmiz1 and included F2460 miz-1 forward primer and F2461 miz-1 reverse primer described earlier, generating the characteristic 2.6 kbp *miz-1* PCR fragment. The third primer pair was F2460 miz-1 forward primer described earlier and F3000 sat reverse primer (GGC TAG TTA GGC GTC ATC CTG A) (seq. Id No. 6) generating a characteristic 4 kbp PCR fragment encompassing the *sat* and *miz-1* genes.

To confirm the integrated construct on the nucleotide level, the *miz-1* gene with its flanking regions was PCR amplified from genomic DNA of recombinant *L. tarentolae* ::plRmiz1 cells using the primer pair F2718 pIR-BgIII-forw. described earlier and F2719 pIR-BgIII-rev. (GGC CGA TTC ATT AAT GCA GGA C) (Seq. ID No. 7) flanking the BgIII site of pIRmiz1. The 3 kbp PCR product was sequenced and found to match the predicted nucleotide sequence.

### Culture of Leishmania tarentolae

*L. tarentolae* was grown in BHI broth (Difco) supplemented with 5 µg/ml hemin. Cells were cultivated either in static cultures using 24 well plates or in actively mixed cultures using 2 liter flasks. The shaking rate was set to 50 rpms or lower. The handling of the cultures was done according to the standard methods described in (Alfonzo et al., 1998) and references therein.

### Identification of the Miz-1 protein:

Recombinant *L*. *tarentolae* ::pIAmiz1 cells were inoculated in 10 ml BHI broth with 5 µg/ml hemin and incubated at 26°C. 2 ml of growing culture were harvested at OD₆₀₀ reading of 1.0 by centrifugation (1 min at 10 000 x g), the cell pellet was lysed in 200 µl of Laemmli sample buffer and protein electrophoresis was performed with 10 µl samples on a 10% SDS-PAGE gel according to standard procedures. The Miz-1 protein was identified by Western blotting with monoclonal antibodies against the hexahistidin tag introduced at the N' terminus of Miz-1 (Qiagen cat.-No. 34610) and with polyclonal rabbit antibodies developed against purified Miz-1.

The results are shown in Fig. 3 A. Column T show the total lysate, Column P show the 1% TritonX-100 insoluble fraction and Column S show the 1% TritonX-100 soluble fraction of *L*. *tarentolae* cells expressing miz. Column M show the molecular weight markers, Column C show the lysate of control cells.

### Example 2

Example of human cytokine Erythropoetin(Epo) with from the episomal plasmid.

Construction of Leishmania tarentolae T7 RNA-Polymerase (T7 RNAP) strain.

A vector pT724 containing T7 RNA polymerase was constructed by excising the T7RNAP gene fused to nuclear localization signal of SV40 virus from pLEW13 plasmid (Misslitz A. et al., ; Tobin and Wirth, 1992; Wirtz et al., 1999) with BgIII and BamHI sites and subcloning it into the Bgl II site of the pIR-SAT vector using standard molecular biological techniques. The integrity of the open reading frame was verified by sequencing. *Leishmania tarentolae* was transformed with the resulting vector as described in the example 1 and the drug resistant clones were selected. The presence of T7RNAP protein was identified in total lysates of transformed cells by Western blotting with policional antibodies against the T7RNAP.

### Construction of pIR-SAT and T7RNAP driven expression of human erithropoetin.

The pIR expression vector used was described in the example 1 but the streptothricin acetyltransferase encoding gene was replaced with hygromycin phosphotransferase and a T7 promoter was introduced at the 5' of the SSU region. The full length coding sequence of human erithropoetin precursor containing signal sequence (EMBL accession Nr.; X02158) was amplified by PCR according to standard procedures from a plasmid source (pcDNA3.1/GS-epo, Invitrogen) with primer containing sequences for the restriction enzyme BamHI at both ends of the primer. Standard molecular cloning techniques were applied to insert the *Epo* gene into the vector pIR-Hyg into BgIII site. Identification of the positive clones was performed as above. The resulting plasmid was elctroporated into the *Leishmania tarentolae* T7 RNAP strain as described in the example 1 with the exemption that no linearisation was performed ensuring that DNA is retained episomaly. Positive clones were selected as described in the example 1 but 50µg/ ml⁻¹ hygromycin was used for the selection. Resistant clones were selected, expended in the BHI medium with 50µg /ml⁻¹ hygromycin and cells as well as cultural supernatants were analyzed by Western bloting with specific polyclonal antibodies. The results are shown in Figure 3 B. The columns T show that supernatants of the cultures contain proteins which are positively reacting with the polyclonal antibodies. The protein recovered in the supernatant migrated slowly. This behavior was attributed to the posttranslational glycosylation typical for Epo and was confirmed by de-glycosilation with nuromidase that resulted in emergence of a smaller species. Column M (Fig. 3 B) show the molecular weight markers, Column C show the lysate of control cells.

### Reference List

Adoutte, A. and Philippe, H. (1993). The major lines of metazoan evolution: summary of traditional evidence and lessons from ribosomal RNA sequence analysis. EXS. 63, 1-30.
Alfonzo, J.D., Thiemann, O.H., and Simpson, L. (1998). Purification and characterization of MAR1. A mitochondrial associated ribonuclease from Leishmania tarentolae. J.Biol.Chem. 273, 30003-30011.
Barry, J.D., Graham, S.V., Fotheringham, M., Graham, V.S., Kobryn, K., and Wymer, B. (1998). VSG gene control and infectivity strategy of metacyclic stage Trypanosoma brucei. Mol.Biochem.Parasitol. 91,93-105.
Beverley, S.M. (1996). Hijacking the cell: parasites in the driver's seat. Cell 87, 787-789.
Beverley, S.M. and Clayton, C.E. (1993). Transfection of Leishmania and Trypanosoma brucei by electroporation. Methods Mol.Biol. 21, 333-348.
Biebinger, S., Wirtz, L.E., Lorenz, P., and Clayton, C. (1997). Vectors for inducible expression of toxic gene products in bloodstream and procyclic Trypanosoma brucei. Mol.Biochem.Parasitol. 85, 99-112.
Blackburn, E.H. (1991). Structure and function of telomeres. Nature 350, 569-573.
Borst, P. and Rudenko, G. (1994). Antigenic variation in African trypanosomes. Science 264, 1872-1873.
Coburn, C.M., Otteman, K.M., McNeely, T., Turco, S.J., and Beverley, S.M. (1991). Stable DNA transfection of a wide range of trypanosomatids. Mol.Biochem.Parasitol. 46,169-179.
Cruz. A., Coburn, C.M., and Beverley, S.M. (1991). Double targeted gene replacement for creating null mutants. Proc.Natl.Acad.Sci.U.S A. 88, 7170-7174.
Elwasila, M. (1988). Leishmania tarentolae Wenyon, 1921 from the gecko Tarentola annularis in the Sudan. Parasitol.Res. 74, 591-592.
Ha, D.S., Schwarz, J.K., Turco, S.J., and Beverley, S.M. (1996). Use of the green fluorescent protein as a marker in transfected Leishmania. Mol.Biochem.Parasitol. 77, 57-64.
Hill, J.O. and Fahey, J.R. (1987). Leishmania spp.: agar plating as an alternative to limiting dilution and impression smears for the enumeration of viable parasites in tissue. Exp.Parasitol. 63, 108-111.
Hotz, H.R., Biebinger, S., Flaspohler, J., and Clayton, C. (1998). PARP gene expression: control at many levels. Mol.Biochem.Parasitol. 91, 131-143.
Hubel A. and Beverley, S. M. A high-level chromosomal expression vector for Leishmania. Materials of the 2nd colloquium on Trypanosoma and Leishmania Research in Germany , 9. 26-3-1999. ZMBH, Heidelberg.
Kapler, G.M., Coburn, C.M., and Beverley, S.M. (1990). Stable transfection of the human parasite Leishmania major delineates a 30-kilobase region sufficient for extrachromosomal replication and expression. Mol.Cell Biol, 10, 1084-1094.
Krakow, J.L., Hereld, D., Bangs, J.D., Hart, G.W., and Englund, P.T. (1986). Identification of a glycolipid precursor of the Trypanosoma brucei variant surface glycoprotein. J.Biol.Chem. 261, 12147-12153.
La Flamme, A.C., Buckner, F.S., Swindle, J., Ajioka, J., and Van Voorhis, W.C. (1996). Trypanosoma cruzi: expression of interleukin-2 utilizing both supercoiled plasmids and linear DNAs. Exp.Parasitol. 83, 159-163.
LeBowitz, J.H., Coburn, C.M., McMahon-Pratt, D., and Beverley, S.M. (1990). Development of a stable Leishmania expression vector and application to the study of parasite surface antigen genes. Proc.Natl.Acad.Sci.U.S A. 87, 9736-9740.
Lu, H.Y. and Buck, G.A. (1991). Expression of an exogenous gene in Trypanosoma cruzi epimastigotes. MoI.Biochem.Parasitol. 44, 109-114.
Melo, M.N. (1981). Cultivation of Leishmania in chemically defined media. Trans.R.Soc.Trop.Med.Hyg. 75, 756
Misslitz A., Mottram J., Overath P., and Aebischer T. Stable (1999) high level expression of betta-galactosidase and GFP in amastigotes of Leishmania mexicana. Abstracts of the 2nd colloquium on trypanosoma and leishmania research in Germany , 13-13
Myler, P.J., Audleman, L., deVos, T., Hixson, G., Kiser, P., Lemley, C., Magness, C., Rickel, E., Sisk, E., Sunkin, S., Swartzell, S., Westlake, T., Bastien, P., Fu, G., Ivens, A., and Stuart, K. (1999). Leishmania major Friedlin chromosome 1 has an unusual distribution of protein-coding genes. Proc.Natl.Acad.Sci.U.S A. 96, 2902-2906.
Papadopoulou, B., Roy, G., and Ouellette, M. (1994). Autonomous replication of bacterial DNA plasmid oligomers in Leishmania. Mol.Biochem.Parasitol. 65, 39-49.
Patnaik, P.K., Bellofatto, V., Hartree, D., and Cross, G.A. (1994). An episome of Trypanosoma brucei can exist as an extrachromosomal element in a broad range of trypanosomatids but shows different requirements for stable replication. Mol.Biochem.Parasitol. 66, 153-156.
Perry, K. and Agabian, N. (1991). mRNA processing in the Trypanosomatidae. Experientia 47, 118-128.
Ray, D.S. and Hines, J.C. (1995). Disruption of the Crithidia fasciculata RNH1 gene results in the loss of two active forms of ribonuclease H. Nucleic.Acids.Res. 23,2526-2530.
Sbicego, S., Schnaufer, A., and Blum, B. (1998). Transient and stable transfection of Leishmania by particle bombardment. Mol.Biochem.Parasitol. 94, 123-126.
Stuart, K. (1991). RNA editing in kinetoplastid protozoa. Curr.Opin.Genet.Dev. 1, 412-416.
Teixeira, S.M. (1998). Control of gene expression in Trypanosomatidae. Braz.J.Med.Biol.Res. 31, 1503-1516.
ten Asbroek, A.L., Mol, C.A., Kieft, R., and Borst, P. (1993). Stable transformation of Trypanosoma brucei. Mol.Biochem.Parasitol. 59, 133-142.
Tobin, J.F. and Wirth, D.F. (1992). A sequence insertion targeting vector for Leishmania enriettii. J.Biol.Chem. 267, 4752-4758.
Wirtz, E., Hartmann, C., and Clayton, C. (1994). Gene expression mediated by bacteriophage T3 and T7 RNA polymerases in transgenic trypanosomes. Nucleic.Acids.Res. 22, 3887-3894.
Wirtz, E., Leal, S., Ochatt, C., and Cross, G.A. (1999). A tightly regulated inducible expression system for conditional gene knock-outs and dominant-negative genetics in Trypanosoma brucei [In Process Citation]. Mol.Biochem.Parasitol. 99, 89-101.
Zhang, W.W., Charest, H., and Matlashewski, G. (1995). The expression of biologically active human p53 in Leishmania cells: a novel eukaryotic system to produce recombinant proteins. Nucleic.Acids.Res. 23, 4073-4080.

## Claims

1. An expression and delivery system comprising a non pathogenic *L. tarentolae* host and a nucleic acid sequence encoding a heterologous protein operably linked to a promoter and wherein the heterelogous nucleic acid sequence is flanked by 5' and 3' UTRs of an actively transcribed *L. tarentolae* host gene.

2. An expression and delivery system of claim 1 wherein the heterologous nucleic acid sequence is flanked by signal sequences for efficient secretion, trans-splicing and polyadenylation of an actively transcribed *Kinetoplastidae* gene.

3. An expression and delivery system of claim 1 to 2 wherein the heterologous nucleic acid sequence is incorporated in a linear or circular vector or plasmid.

4. An expression and delivery system of claim 1 to 3 wherein two or more vectors or plasmids are incorporated.

5. An expression and delivery system of claim 1 to 4 wherein the heterologous protein is an enzyme selected from the group consisting of catalase, laccase, phenoloxidase, oxidase, oxidoreductases, cellulase xylanase, peroxidase, lipase, hydrolase, esterase, cutinase, protease and other proteolytic enzymes, aminopeptidase, carboxypeptidase, phytase, lyase, pectinase and other pectinolytic enzymes, amylase glucoamylase, α-galactosidase, β-gatactosidase, α-glucosidase, β-glucosidase, mannosidase, isomerase, invertase, transferase, ribonuclease, chitinase and deoxyribonuclease.

6. An expression and delivery system of claim 1 to 5, wherein the non pathogenic *L*. *tarentolae* host or the incorporated vector or plasmid comprises a selectable marker gene.

7. An expression and delivery system of claim 6 wherein the marker gene is selected from the group consisting of hygromycin phosphotransferase (HYG) gene in combination with hygromycin, neomycin phosphotransferase (NEO) gene in combination with G418, product of the *Streptoalloteichus hindustanus* BLE gene (BLE) in combination with phleomycin and streptothricin acetyltransferase encoding (SAT) gene in combination with nourseothricin.

8. An expression and delivery system of claim 1 to 7 wherein the promoter is an actively transcribed Kinetoplastidae gene promotor or a strongly transcription initiating heterologous promotor.

9. An expression and delivery system of claim 1 to 8 wherein the transcription of the heterologous nucleic acid is controlled by an repressor responsive element in connection with an incorporated and expressed repressor gene.

10. An expression and delivery system of claim 1 to 9 wherein at least one copy of the heterologous nucleic acid is located in an actively transcribed gene cluster of the *L. tarentolae* host cell.

11. An expression and delivery system of claim 10 wherein the actively transcribed gene cluster is a rRNA gene cluster.

12. An expression and delivery system of claim 1 to 9 wherein the heterologous nucleic acid is located in an episomally transcribed plasmid.

13. A vector or plasmid comprising the heterologous nucleic acid prefaced of a promotor and flanked by 5' and 3' UTRs of an actively transcribed *L*. *tarentolae* host gene.

14. A vector or plasmid of claim 13 comprising signal sequences for efficient secretion, trans-splicing and/or polyadenylation of an actively transcribed *Kinetoplastidae* gene.

15. A vector or plasmid of claim 13 or 14 comprising a selection marker gene.

16. Non pathogenic *L. tarentolae* cell lines of claim 1 which are stable transformed with the heterologous nucleic acid.

17. Non pathogenic *L*. *tarentolae* cell lines of claim 1 which are stable transformed with the heterologous nucleic acid comprising vectors or plasmids of claim 13 to 15.

18. A method for producing the heterologous protein in *L. tarentolae* host cells of claim 1 wherein a stable transformed non pathogenic *L. tarentolae* cell line comprising
a) a DNA sequence coding for a gene of a selectable marker and
b) a heterologous DNA sequence coding for a desired protein operably linked and integrated into the actively transcribed gene,
is cultivated on selection media for constitutive heterologous gene expression.

19. A method for producing the heterologous protein in *L. tarentolae* host cells of claim 1 wherein a stable transformed non pathogenic *L. tarentolae* cell line comprising,
a) a DNA sequence coding for a selectable marker gene and
b) the heterologous DNA sequence coding for a desired protein operably linked into an episomally maintained plasmid DNA with an active promotor,
is cultivated on selection media for constitutive heterologous gene expression.

20. A method for producing a heterologous protein in *L. tarentolae* host cells of claim 1 wherein a stable transformed non pathogenic *L. tarentolae* cell line comprising
a) a DNA sequence coding for a heterologous RNA polymerase, integrated into an actively transcribed gene cluster and
b) a DNA sequence coding for a selectable marker integrated into an actively transcribed gene cluster and
c) a DNA sequence coding for a transcription repressor gene integrated into an actively transcribed gene cluster and
d) a heterologous DNA sequence coding for a desired protein prefaced with the heterologous RNA polymerase promoter and a repressor response element, is cultivated with a selectable marker and the expression of the heterologous gene is induced with an inhibitor of the heterologous repressor.

21. A method of claim 20 wherin the heterologous RNA polymerase is a T7 or T3 RNA polymerase.

22. A method of claim 20 wherin the repressor is a heterologous Tet or Lac repressor.

23. The use of a expression and / or delivery system of claim 1 for the expression of heterologous proteins.

24. The use of a expression and / or delivery system of claim 1 for the delivery of heterologous proteins into plant cells or animal cells by transfection with the non pathogenic *L. tarentolae* host.

25. The use of a expression and / or delivery system of claim 1 for the expression and/or delivery of heterologous eucaryotic proteins.

## Patentansprüche

1. Expressions- und Zufuhrsystem, umfassend einen nicht-pathogenen *L.-tarentolae*-Wirt und eine Nukleinsäuresequenz, die ein heterologes Protein codiert und operativ mit einem Promotor verbunden ist, und wobei die heterologe Nukleinsäuresequenz von 5'-und 3'-UTRs eines aktiv transkribierten *L.-tarentolae-*Wirtsgens flankiert ist.

2. Expressions- und Zufuhrsystem nach Anspruch 1, wobei die heterologe Nukleinsäuresequenz von Signalsequenzen für eine effiziente Sekretion, Trans-Spleißen und Polyadenylierung eines aktiv transkribierten Kinetoplastidae-Gens flankiert ist.

3. Expressions- und Zufuhrsystem nach Anspruch 1 bis 2, wobei die heterologe Nukleinsäuresequenz in einen linearen oder zirkulären Vektor oder ein lineares oder zirkuläres Plasmid eingebracht wird.

4. Expressions- und Zufuhrsystem nach Anspruch 1 bis 3, wobei zwei oder mehr Vektoren oder Plasmide eingebracht werden.

5. Expressions- und Zufuhrsystem nach Anspruch 1 bis 4, wobei das heterologe Protein ein Enzym ist, ausgewählt aus der Gruppe, die aus Katalase, Laccase, Phenoloxidase, Oxidase, Oxidoreduktasen, Cellulase, Xylanase, Peroxidase, Lipase, Hydrolase, Esterase, Cutinase, Protease und anderen proteolytischen Enzymen, Aminopeptidase, Carboxypeptidase, Phytase, Lyase, Pektinase und anderen pektinolytischen Enzymen, Amylase, Glucoamylase, α-Galactosidase, β-Galactosidase, α-Glucosidase, β-Glucosidase, Mannosidase, Isomerase, Invertase, Transferase, Ribonuklease, Chitinase und Desoxyribonuklease besteht.

6. Expressions- und Zufuhrsystem nach Anspruch 1 bis 5, wobei der nicht-pathogene *L.-tarentolae*-Wirt oder der eingebrachte Vektor oder das eingebrachte Plasmid ein selektierbares Markergen umfasst.

7. Expressions- und Zufuhrsystem nach Anspruch 6, wobei das Markergen aus der Gruppe ausgewählt ist, die aus einen Hygromycin-Phosphotransferase- (HYG-) Gen in Kombination mit Hygromycin, einem Neomycin-Phosphotransferase- (NEO-) Gen in Kombination mit G418, dem Produkt des *Streptoalloteichus-hindustanus*-BLE-Gens (BLE) in Kombination mit Phleomycin und dem Streptothricin-Acetyltransferase codierenden Gen (SAT) in Kombination mit Nourseothricin besteht.

8. Expressions- und Zufuhrsystem nach Anspruch 1 bis 7, wobei der Promotor ein Promotor eines aktiv transkribierten Kinetoplastidae-Gens oder ein stark die Transkription initiierender heterologer Promotor ist.

9. Expressions- und Zufuhrsystem nach Anspruch 1 bis 8, wobei die Transkription der heterologen Nukleinsäure durch ein Repressor-responsives Element in Verbindung mit einem eingebrachten und exprimierten Repressorgen gesteuert wird.

10. Expressions- und Zufuhrsystem nach Anspruch 1 bis 9, wobei mindestens eine Kopie der heterologen Nukleinsäure sich in einem aktiv transkribierten Gen-Cluster der *L.-tarentolae*-Wirtszelle befindet.

11. Expressions- und Zufuhrsystem nach Anspruch 10, wobei das aktiv transkribierte Gen-Cluster ein rRNA-Gen-Cluster ist.

12. Expressions- und Zufuhrsystem nach Anspruch 1 bis 9, wobei die heterologe Nukleinsäure sich in einem episomal transkribierten Plasmid befindet.

13. Vektor oder Plasmid, umfassend die heterologe Nukleinsäure, der ein Promotor vorausgeht und die von 5'- und 3'-UTRs eines aktiv transkribierten L.-*tarentolae-Wirtsgens flankiert* wird.

14. Vektor oder Plasmid nach Anspruch 13, umfassend Signalsequenzen für eine effiziente Sekretion, Trans-Spleißen und/oder Polyadenylierung eines aktiv transkribierten *Kinetoplastidae-*Gens*.*

15. Vektor oder Plasmid nach Anspruch 13 oder 14, umfassend ein Selektionsmarker-Gen.

16. Nicht-pathogene *L.-tarentolae*-Zelllinien nach Anspruch 1, die mit der heterologen Nukleinsäure stabil transformiert sind.

17. Nicht-pathogene *L.-tarentolae*-Zelllinien nach Anspruch 1, die mit der heterologen Nukleinsäure stabil transformiert sind und Vektoren oder Plasmide nach Anspruch 13 bis 15 umfassen.

18. Verfahren zur Produktion des heterologen Proteins in *L.-tarentolae*-Wirtszellen nach Anspruch 1, wobei eine stabil transformierte nicht-pathogene *L.-*tarentolae-Zelllinie, umfassend
a) eine DNA-Sequenz, die für ein Gen eines selektierbaren Markers codiert, und
b) eine heterologe DNA-Sequenz, die für ein gewünschtes Protein codiert, operativ verbunden und integriert in das aktiv transkribierte Gen,
auf Selektionsmedien für die konstitutive heterologe Genexpression kultiviert wird.

19. Verfahren zur Produktion des heterologen Proteins in *L.-tarentolae*-Wirtszellen nach Anspruch 1, wobei eine stabil transformierte nicht-pathogene L.-*tarentolae*-Zelllinie, umfassend
a) eine DNA-Sequenz, die für ein selektierbares Markergen codiert, und
b) eine heterologe DNA-Sequenz, die für ein gewünschtes Protein codiert, operativ verbunden in einer episomal aufrecht erhaltenen Plasmid-DNA mit einem aktiven Promotor,
auf Selektionsmedien für die konstitutive heterologe Genexpression kultiviert wird.

20. Verfahren zur Produktion eines heterologen Proteins in *L.-tarentolae*-Wirtszellen nach Anspruch 1, wobei eine stabil transformierte nicht-pathogene L.-tarentolae-Zelllinie, umfassend
a) eine DNA-Sequenz, die für eine heterologe RNA-Polymerase codiert, integriert in ein aktiv transkribiertes Gen-Cluster, und
b) eine DNA-Sequenz, die für einen selektierbaren Marker codiert, integriert in ein aktiv transkribiertes Gen-Cluster, und
c) eine DNA-Sequenz, die für ein Transkriptionsrepressor-Gen codiert, integriert in ein aktiv transkribiertes Gen-Cluster, und
d) eine heterologe DNA-Sequenz, die für ein gewünschtes Protein codiert, der der heterologe RNA-Polymerase-Promotor und ein Repressor-responsives Element vorausgehen,
mit einem selektierbaren Marker kultiviert wird und die Expression des heterologen Gens mit einem Inhibitor des heterologen Repressors induziert wird.

21. Verfahren nach Anspruch 20, wobei die heterologe RNA-Polymerase T7- oder T3-RNA-Polymerase ist.

22. Verfahren nach Anspruch 20, wobei der Repressor ein heterologer Tet- oder Lac-Repressor ist.

23. Verwendung eines Expressions- und/oder Zufuhrsystems nach Anspruch 1 zur Expression heterologer Proteine.

24. Verwendung eines Expressions- und/oder Zufuhrsystems nach Anspruch 1 zur Zufuhr heterologer Proteine in Pflanzenzellen oder Tierzellen durch Transfektion mit dem nicht-pathogenen *L.-tarentolae-*Wirt.

25. Verwendung eines Expressions- und/oder Zufuhrsystems nach Anspruch 1 zur Expression und/oder Zufuhr heterologer eukaryotischer Proteine.

## Revendications

1. Système d'expression et d'administration comprenant un hôte *L*. *tarentolae* non pathogène et une séquence d'acide nucléique codant pour une protéine hétérologue liée de manière fonctionnelle à un promoteur et dans lequel la séquence d'acide nucléique hétérologue est flanquée par les régions 3'-UTR et 5'-UTR d'un gène activement transcrit de l'hôte *L. tarentolae.*

2. Système d'expression et d'administration selon la revendication 1, dans lequel la séquence d'acide nucléique hétérologue est flanquée par des séquences signal pour une sécrétion, un épissage en trans et une polyadénylation efficaces d'un gène activement transcrit de *Kinetoplastidae.*

3. Système d'expression et d'administration selon la revendication 1 ou 2, dans lequel la séquence d'acide nucléique hétérologue est incorporée dans un vecteur ou plasmide linéaire ou circulaire.

4. Système d'expression et d'administration selon les revendications 1 à 3, dans lequel deux vecteurs ou plasmides ou plus sont incorporés.

5. Système d'expression et d'administration selon les revendications 1 à 4, dans lequel la protéine hétérologue est une enzyme choisie dans le groupe constitué par la catalase, la laccase, la phénoloxydase, l'oxydase, les oxydoréductases, la cellulase xylanase, la peroxydase, la lipase, l'hydrolase, l'estérase, la cutinase, la protéase et d'autres enzymes protéolytiques, l'aminopeptidase, la carboxypeptidase, la phytase, la lyase, la pectinase et d'autres enzymes pectinolytiques, l'amylase glucoamylase, l' α-galactosidase, la β-galactosidase, l'α-glucosidase, la β-glucosidase, la mannosidase, l'isomérase, l'invertase, la transférase, la ribonucléase, la chitinase et la désoxyribonucléase.

6. Système d'expression et d'administration selon les revendications 1 à 5, dans lequel l'hôte *L*. *tarentolae* non pathogène ou le vecteur ou plasmide incorporé comprend un gène marqueur sélectionnable.

7. Système d'expression et d'administration selon la revendication 6, dans lequel le gène marqueur est choisi dans le groupe constitué par le gène de l'hygromycine phosphotransférase (HYG) combiné à de l'hygromycine, par le gène de la néomycine phosphotransférase (NEO) combiné à du G418, par le produit du gène BLE de *Streptoalloteichus hindustanus* (BLE) combiné à de la phléomycine et par le gène codant pour la streptothricine acétyltransférase (SAT) combiné à de la nourséothricine.

8. Système d'expression et d'administration selon les revendications 1 à 7, dans lequel le promoteur est un promoteur de gène activement transcrit de *Kinetoplastidae* ou un promoteur hétérologue initiant fortement la transcription.

9. Système d'expression et d'administration selon les revendications 1 à 8, dans lequel la transcription de l'acide nucléique hétérologue est contrôlée par un élément réagissant avec un répresseur conjointement avec un gène répresseur incorporé et exprimé.

10. Système d'expression et d'administration selon les revendications 1 à 9, dans lequel au moins une copie de l'acide nucléique hétérologue est située dans une batterie de gènes activement transcrits de la cellule de l'hôte *L*. *tarentolae.*

11. Système d'expression et d'administration selon la revendication 10, dans lequel la batterie de gènes activement transcrits est une batterie de gènes d'ARNr.

12. Système d'expression et d'administration selon les revendications 1 à 9, dans lequel l'acide nucléique hétérologue est situé dans un plasmide transcrit de façon épisomique.

13. Vecteur ou plasmide comprenant l'acide nucléique hétérologue précédé d'un promoteur et flanqué par les régions 5'-UTR et 3'-UTR d'un gène activement transcrit de l'hôte *L. tarentolae.*

14. Vecteur ou plasmide selon la revendication 13, comprenant des séquences signal pour une sécrétion, un épissage en trans et une polyadénylation efficaces d'un gène activement transcrit de *Kinetoplastidae.*

15. Vecteur ou plasmide selon la revendication 13 ou 14, comprenant un gène marqueur de sélection.

16. Lignées de cellules de *L. tarentolae* non pathogène selon la revendication 1 qui sont transformées de façon stable avec l'acide nucléique hétérologue.

17. Lignées de cellules de *L. tarentolae* non pathogène selon la revendication 1, qui sont transformées de façon stable avec l'acide nucléique hétérologue comprenant les vecteurs ou plasmides selon les revendications 13 à 15.

18. Procédé pour produire la protéine hétérologue dans des cellules de l'hôte *L. tarentolae* selon la revendication 1, dans lequel une lignée de cellules de *L*. *tarentolae* non pathogène transformée de façon stable comprenant :
a) une séquence d'ADN codant pour un gène d'un marqueur sélectionnable et
b) une séquence d'ADN hétérologue codant pour une protéine souhaitée liée de manière fonctionnelle et intégrée dans le gène activement transcrit,
est cultivée sur du milieu de sélection pour l'expression constitutive de gènes hétérologues.

19. Procédé pour produire la protéine hétérologue dans des cellules hôtes de *L. tarentolae* selon la revendication 1, dans lequel une lignée de cellules de *L*. *tarentolae* non pathogène transformée de façon stable comprenant :
a) une séquence d'ADN codant pour un gène d'un marqueur sélectionnable et
b) une séquence d'ADN hétérologue codant pour une protéine souhaitée liée de manière fonctionnelle dans un ADN plasmidique conservé de façon épisomique à un promoteur actif,
est cultivée sur du milieu de sélection pour l'expression constitutive de gènes hétérologues.

20. Procédé pour produire une protéine hétérologue dans des cellules hôtes de *L. tarentolae* selon la revendication 1, dans lequel une lignée de cellules de *L*. *tarentolae* non pathogène transformée de façon stable comprenant :
a) une séquence d'ADN codant pour une ARN polymérase hétérologue, intégrée dans une batterie de gènes activement transcrits et
b) une séquence d'ADN codant pour un marqueur sélectionnable intégré dans une batterie de gènes activement transcrits et
c) une séquence d'ADN codant pour un gène répresseur de la transcription intégré dans une batterie de gènes activement transcrits et
d) une séquence d'ADN hétérologue codant pour une protéine souhaitée précédée du promoteur de l'ARN polymérase hétérologue et d'un élément réagissant avec un répresseur,
est cultivée avec un marqueur sélectionnable et l'expression du gène hétérologue est induite par un inhibiteur du répresseur hétérologue.

21. Procédé selon la revendication 20, dans lequel l'ARN polymérase hétérologue est une T7 ARN polymérase ou une T3 ARN polymérase.

22. Procédé selon la revendication 20, dans lequel le répresseur est un répresseur Tet ou Lac hétérologue.

23. Utilisation d'un système d'expression et/ou d'administration selon la revendication 1 pour l'expression de protéines hétérologues.

24. Utilisation d'un système d'expression et/ou d'administration selon la revendication 1 pour l'administration de protéines hétérologues dans des cellules végétales ou dans des cellules animales par transfection avec l'hôte *L. tarentolae* non pathogène.

25. Utilisation d'un système d'expression et/ou d'administration selon la revendication 1 pour l'expression et/ou l'administration de protéines eucaryotes hétérologues.
